(19) Europäisches Patentamt
European Patent Office
Office européen des brevets

(11) Veröffentlichungsnummer: **0 187 376 B1**

(12) # EUROPÄISCHE PATENTSCHRIFT

(45) Veröffentlichungstag der Patentschrift: **13.05.92**

(21) Anmeldenummer: **85116551.4**

(22) Anmeldetag: **24.12.85**

Die Akte enthält technische Angaben, die nach dem Eingang der Anmeldung eingereicht wurden und die nicht in dieser Patentschrift enthalten sind.

(51) Int. Cl.[5]: **C07D 471/04**, A61K 31/435, A61K 31/495, C07D 213/61, C07D 213/77, C07D 213/78, //C07D213/80,(C07D471/04, 221:00,221:00)

(54) **6,7-Disubstituierte 1-Cyclopropyl-1,4-dihydro-4-oxo-1,8-naphthyridin-3-carbonsäuren.**

(30) Priorität: **10.01.85 DE 3500562**
**13.03.85 DE 3508816**

(43) Veröffentlichungstag der Anmeldung:
**16.07.86 Patentblatt 86/29**

(45) Bekanntmachung des Hinweises auf die Patenterteilung:
**13.05.92 Patentblatt 92/20**

(84) Benannte Vertragsstaaten:
**AT BE CH DE FR GB IT LI NL SE**

(56) Entgegenhaltungen:
**EP-A- 27 752** **EP-A- 78 362**
**EP-A- 113 093** **EP-A- 117 473**
**EP-A- 0 004 279** **EP-A- 0 049 355**
**EP-A- 0 106 489** **EP-A- 0 132 845**
**EP-A- 0 153 163** **EP-A- 0 153 828**
**EP-A- 0 160 578** **EP-A- 0 205 029**
**EP-A- 0 207 420**

**Chemical Abstracts, Band 103, 1985, Seite 581, Zusammenfassung, 22617d, Columbus, Ohio, US; & JP-A-6032790 (Dainippon Pharm.Co.Ltd.) 19.02.1985., Zusammenfassung.**

(73) Patentinhaber: **BAYER AG**

**W-5090 Leverkusen 1 Bayerwerk(DE)**

(72) Erfinder: **Petersen, Uwe, Dr.**
**Auf dem Forst 4**
**W-5090 Leverkusen 1(DE)**
Erfinder: **Grohe, Klaus, Dr.**
**Am Wasserturm 10**
**W-5068 Odenthal(DE)**
Erfinder: **Zeiler, Hans-Joachim, Dr.**
**Am Rohm 86**
**W-5600 Wuppertal 1(DE)**
Erfinder: **Metzger, Karl Georg, Dr.**
**Pahlkestrasse 75**
**W-5600 Wuppertal 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.08/2.19/2.0)

## Beschreibung

Die vorliegende Erfindung betrifft neue 6,7-disubstituierte 1-Cyclopropyl-1,4-dihydro-4-oxo-1,8-naphthyridin-3-carbonsäuren, Verfahren zu ihrer Herstellung sowie diese Verbindungen enthaltende antibakterielle Mittel und Futterzusatzstoffe.

Aus der EP-A- 0 106 489, 0 049 355, 0 004 279, 0 153 828, 0 160 578, 0 132 845, 0 153 163 sind bereits Wirkstoffe vom 1,8-Naphthyridon-3-carbonsäure-Typ bekannt geworden, deren Wirkstärke und -spektrum jedoch nicht in allen Bereichen zufriedenstellend ist.

Es wurde gefunden, daß die neuen 6,7-disubstituierten 1-Cyclopropyl-1,4-dihydro-4-oxo-1,8-naphthyridin-3-carbonsäuren der Formel (I) in welcher

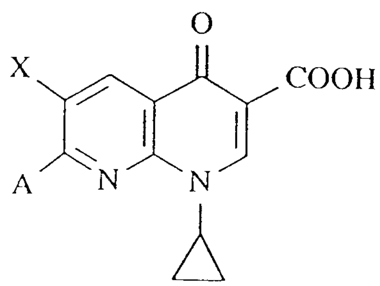

(I)

X für Halogen und

A für

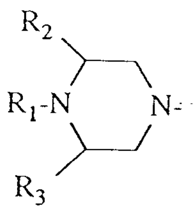

oder

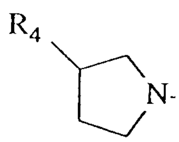

stehen

worin

$R_1$ für verzweigtes oder geradkettiges Alkyl mit 1 - 4 Kohlenstoffatomen steht, das durch eine Hydroxy- oder Ethoxygruppe substituiert sein kann, einen gegebenenfalls durch Hydroxy, Methoxy, Chlor oder Fluor substituierten Phenacylrest, Oxopropyl, 2-Oxobutyl oder für den Rest

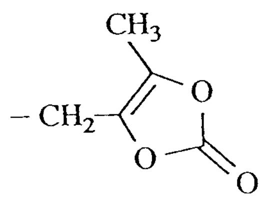

steht,

$R_2$ für Wasserstoff oder Methyl oder gegebenenfalls durch Chlor, Fluor, Methyl, Hydroxy oder Methoxy substituiertes Phenyl oder Thienyl,

$R_3$ für Wasserstoff oder Methyl oder

$R_4$ für Hydroxymethyl steht,

sowie deren pharmazeutisch verwendbare Hydrate, Säureadditionssalze, Alkali-, Erdalkali-, Silber- und Guanidiniumsalzen sowie ihren Methyl-, Ethyl-, Pivaloyloxymethyl-, Pivaloyloxyethyl oder deren (5-Methyl-2-oxo-1,3-dioxol-4-yl-methyl)ester, wobei $R_1$ nicht Methyl sein kann wenn X Fluor bedeutet

und deren pharmazeutisch verwendbare Hydrate, Säureadditionssalze, Alkali-, Erdalkali, Silber- und Guanidiniumsalze, sowie in Form ihrer Ester und in anderen üblichen Prodrug-Formen eine hohe antibakterielle Wirkung aufweisen.

Sie eignen sich daher als Wirkstoffe für die Human- und Veterinärmedizin, wobei zur Veterinärmedizin auch die Behandlung von Fischen zur Therapie oder Vorbeugung bakterieller Infektionen zu zählen ist.

Bevorzugt sind diejenigen Verbindungen der Formel (I), in denen

X für Fluor oder Chlor und

A für

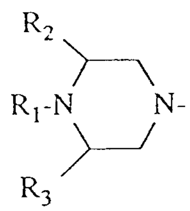

oder

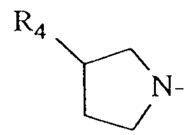

stehen

worin

$R_1$ für verzweigtes oder geradkettiges Alkyl mit 1 - 4 Kohlenstoffatomen steht, das durch eine Hydroxy- oder Ethoxygruppe substituiert ist, einen durch Hydroxy, Methoxy, Chlor oder Fluor substituierten Phenacylrest, 2-Oxopropyl, 2-Oxobutyl oder für den Rest

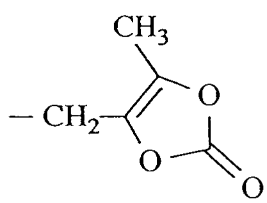

steht,

$R_2$ für Wasserstoff oder Methyl oder gegebenenfalls durch Chlor, Fluor, Methyl, Hydroxy oder Methoxy substituiertes Phenyl oder Thienyl,

$R_3$ für Wasserstoff oder Methyl und

$R_4$ für Hydroxymethyl steht,

sowie deren pharmazeutisch verwendbare Hydrate, Säureadditionssalze, Alkali-, Erdalkali-, Silber- und Guanidiniumsalzen sowie ihren Methyl-, Ethyl-, Pivaloyloxymethyl-, Pivaloyloxyethyl oder deren (5-Methyl-2-oxo-1,3-dioxol-4-yl-methyl)ester, wobei $R_1$ nicht Methyl sein kann wenn X Fluor bedeutet.

Besonders bevorzugt sind diejenigen Verbindungen der Formel (I), in denen

X für Fluor oder Chlor, und

A für

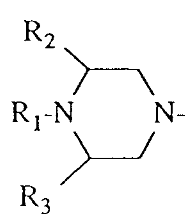

steht,

worin

$R_1$ für verzweigtes oder geradkettiges Alkyl mit 1 - 4 Kohlenstoffatomen steht, das durch eine Hydroxy- oder Ethoxygruppe substituiert ist, einen gegebenenfalls durch Hydroxy, Methoxy, Chlor

oder Fluor substituierten Phenacylrest, 2-Oxopropyl,2-Oxobutyl oder für den Rest

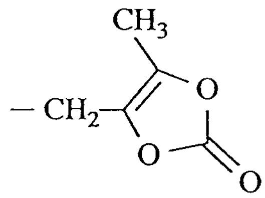

steht,

$R_2$ für Wasserstoff oder Methyl oder gegebenenfalls durch Chlor, Fluor, Methyl, Hydroxy oder Methoxy substituiertes Phenyl oder Thienyl,

$R_3$ für Wasserstoff oder Methyl steht,

sowie deren pharmazeutisch verwendbare Hydrate, Säureadditionssalze, Alkali-, Erdalkali-, Silber- und Guanidiniumsalzen sowie ihren Methyl-, Ethyl-, Pivaloyloxymethyl-, Pivaloyloxyethyl oder deren (5-Methyl-2-oxo-1,3-dioxol-4-yl-methyl)ester, wobei $R_1$ nicht Methyl sein kann wenn X Fluor bedeutet.

Bevorzugt sind im übrigen die Verbindungen der Formel (I) in Form ihrer Methyl-, Ethyl-, Pivaloyloxymethyl-, Pivaloyloxyethyl- oder (5-Methyl-2-oxo-1,3-dioxol-4-yl-methyl)-ester sowie 1-Cyclopropyl-6-fluor-1,4-dihydro-7-(3-methyl-1-piperazinyl)-4-oxo-1,8-naphthyridin-3-carbonsäure und 1-Cyclopropyl-6-fluor-1,4-dihydro-7-(4(2-hydroxyethyl)-1-piperazinyl)-4-oxo-1,8- naphthyridin-3-carbonsäure.

Weiterhin wurde gefunden, daß man die Verbindungen der Formel (I) erhält, wenn man die 1-Cyclopropyl-7-halogen-1,4-dihydro-4-oxo-1,8-naphthyridin-3-carbonsäuren der Formel (II),

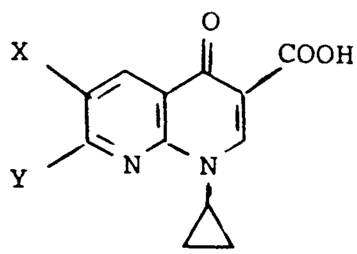

(II)

in welcher

X die oben angegebene Bedeutung hat und

Y für Halogen, bevorzugt Chlor oder Fluor steht, mit Aminen der Formel (III),

A - H (III)

in welcher

A die oben angegebene Bedeutung, ausgenommen Halogen, hat,

gegebenenfalls in Gegenwart von Säurebindern umsetzt (Methode A).

Erfindungsgemäße Verbindungen der Formel (I), wobei $R_1$ = H ausgenommen ist, können auch erhalten werden, indem man 1-Cyclopropyl-1,4-dihydro-4-oxo-7-(1-piperazinyl)-1,8-naphthyridin-3-carbonsäuren der Formel (IV),

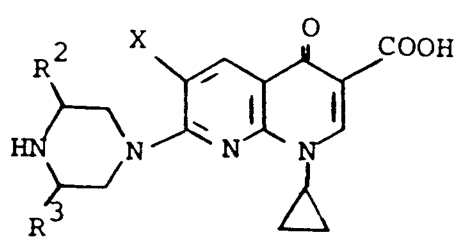

(IV)

in welcher
X, $R^2$ und $R^3$ die oben angegebene Bedeutung haben, mit Verbindungen der Formel (V),

$R^1$ - Z (V)

in welcher
- $R^1$ die oben angegebene Bedeutung hat, jedoch nicht Wasserstoff sein kann, und
- Z Halogen, insbesondere Chlor, Brom oder Jod, Acyloxy, Ethoxy oder Hydroxy

bedeutet,
gegebenenfalls in Gegenwart von Säurebindern umsetzt (Methode B).

Man erhält erfindungsgemäße Verbindungen der Formel (I) ($R^1 = CH_3$-CO-$CH_2CH_2$-) auch, wenn man eine Verbindung der Formel (IV) mit Methylvinylketon der Formel (VI)

$CH_3$ - CO - CH = $CH_2$ (VI)

umsetzt (Methode C).

Verwendet man bei der Umsetzung nach Methode A 2-Methylpiperazin und 7-Chlor-1-cyclopropyl-6-fluor-1,4-dihydro-4-oxo-1,8-naphthyridin-3-carbonsäure als Ausgangsstoffe, so kann der Reaktionsablauf durch das folgende Formelschema wiedergegeben werden:

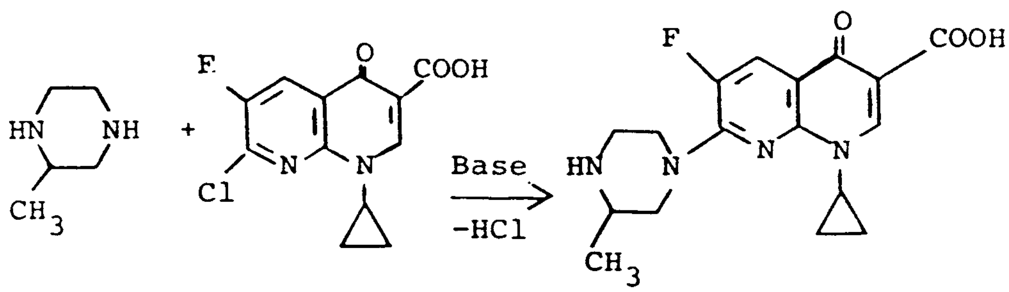


Verwendet man beispielsweise bei der Umsetzung nach Methode B Chloraceton und 6-Chlor-1-cyclopropyl-1,4-dihydro-4-oxo-7-(1-piperazinyl)-1,8-naphthyridin-3-carbonsäure als Ausgangsstoffe, so kann der Reaktionsablauf durch das folgende Formelschema wiedergegeben werden:

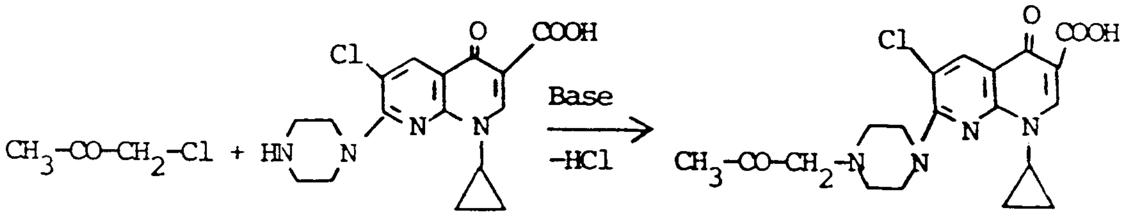


Verwendet man beispielsweise nach Methode C Methylvinylketon und 1-Cyclopropyl-6-fluor-1,4-dihydro-4-oxo-7-(1-piperazinyl)-1,8-naphthyridin-3-carbonsäure als Ausgangsverbindungen, so kann der Reaktionsablauf durch das folgende Formelschema wiedergegeben werden:

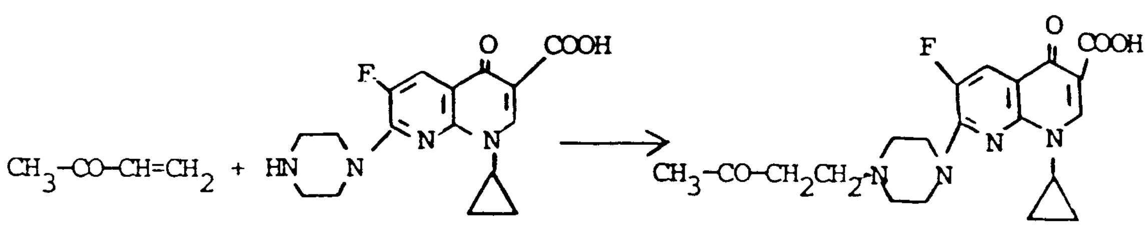

Die als Ausgangsstoffe nach Methode A verwendeten 1-Cyclopropyl-7-halogen-1,4-dihydro-4-oxo-1,8-naphthyridin-3-carbonsäuren der Formel (II) können gemäß folgendem Reaktionsschema hergestellt werden:

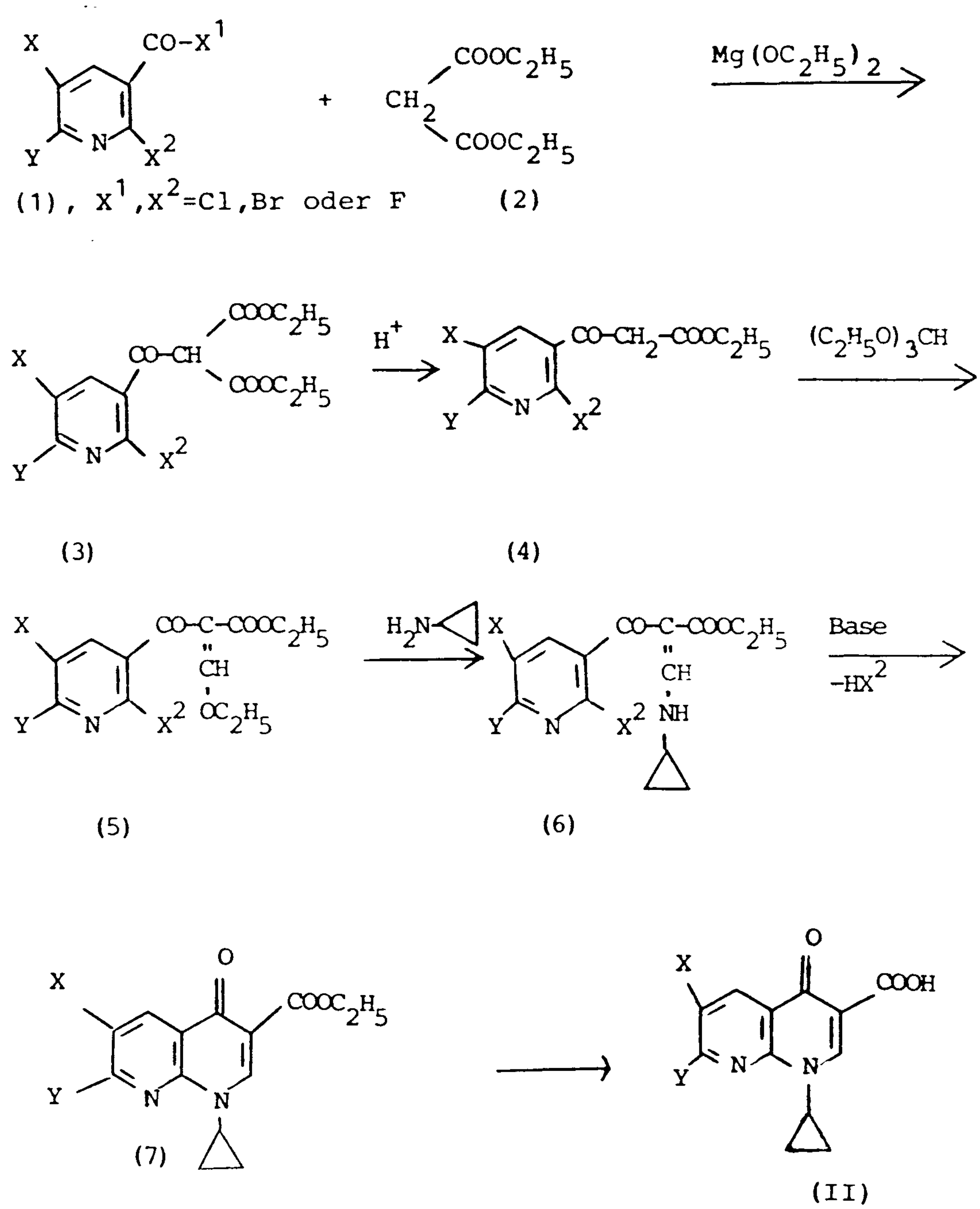


(1), $X^1$,$X^2$=Cl,Br oder F (2)

(3) (4)

(5) (6)

(7) (II)

Danach wird Malonsäurediethylester (2) in Gegenwart von Magnesiumethylat mit dem entsprechenden Nicotinsäurehalogenid (1) zum Acylmalonester (3) acyliert (Organicum, 3. Aufl. 1964, S. 438).

Durch partielle Verseifung und Decarboxylierung von (3) in wäßrigem Medium mit katalytischen Mengen Schwefelsäure oder 4-Toluolsulfonsäure erhält man in guter Ausbeute den Acylessigsäureethylester (4), der mit Orthoameisensäure-triethylester/Acetanhydrid in den 2-(Nicotinoyl)-3-ethoxy-acrylsäureethylester (5) übergeht. Die Umsetzung von (5) mit Cyclopropylamin in einem Lösungsmittel, wie z.B. Methylenchlorid, einem Alkohol, Chloroform, Cyclohexan oder Toluol führt in leicht exothermer Reaktion zum gewünschten Zwischenprodukt (6).

Die Cyclisierungsreaktion (6) → (7) wird in einem Temperaturbereich von etwa 60 bis 300 °C, bevorzugt 80 bis 180 °C durchgeführt.

Als Verdünnungsmittel können Dioxan, Dimethylsulfoxid, N-Methylpyrrolidon, Sulfolan, Hexamethylphosphorsäuretrisamid und bevorzugt N,N-Dimethylformamid verwendet werden.

Als Säurebinder kommen für diese Reaktionsstufe Kalium-tert.-butanolat, Butyl-lithium, Lithium-phenyl, Phenylmagnesiumbromid, Natriummethylat, Natriumhydrid, Natrium- oder Kaliumcarbonat in Betracht. Besonders bevorzugt werden Kalium- oder Natriumfluorid, wenn Fluorwasserstoff abgespalten werden muß. Es kann vorteilhaft sein, einen Überschuß von 10 Mol-% an Base einzusetzen.

Die in dem letzten Schritt erfolgende Esterhydrolyse von (7) unter basischen oder sauren Bedingungen führt zu den 1-Cyclopropyl-7-halogen-1,4-dihydro-4-oxo-1,8-naphthyridin-3-carbonsäuren (II).

Das als Ausgangsstoff für diesen Syntheseweg verwendete 2,5,6-Trichlorpyridin-3-carbonsäurechlorid [Helv. Chim. Acta 59, 222 (1976)] ist bereits bekannt. 2,6-Dichlor-5-fluor-pyridin-3-carbonsäurechlorid kann auf folgendem Wege erhalten werden: 5-Amino-2,6-dichlor-3-methylpyridin [Helv. Chim. Acta 59, 190 (1976)] wird über 2,6-Dichlor-3-methyl-5-(3,3-dimethyl-1-triazeno)-pyridin oder durch eine Baltz-Schiemann-Reaktion in 2,6-Dichlor-5-fluor-3-methyl-pyridin überführt. Dieses wird zu 2,6-Dichlor-5-fluor-3-trichlormethyl-pyridin chloriert. Durch nachfolgende Hydrolyse mit Schwefelsäure erhält man die Carbonsäure, die auf üblichem Wege in 2,6-Dichlor-5-fluor-pyridin-3-carbonsäurechlorid umgewandelt wird. Alternativ hierzu kann man auch 5-Fluor-2,6-dihydroxypyridin-3-carboxamid [J. Amer. Chem. Soc. 101, 4423 (1979); J. Org. Chem. 46, 846 (1981)] mit Phosphoroxichlorid in 2,6-Dichlor-5-fluor-pyridin-3-carbonitril überführen und dieses ebenso nach Verseifung zur Carbonsäure zum Säurechlorid umsetzen. Durch Oxydation von 2,6-Dichlor-3-chlormethyl-5-nitro-pyridin [Helv. Chim. Acta 59, 190 (1976)] erhält man die entsprechende Nicotinsäure, die mit Thionylchlorid das 2,6-Dichlor-5-nitro-pyridin-3-carbonsäurechlorid liefert.

Die als Ausgangsstoffe verwendeten Amine der Formel (III) sind bekannt [US-PS 4 166 180, J. Med. Chem. 26, 1116 (1983)]. Als Beispiele seien genannt: Piperazin, N-Methylpiperazin, N-Ethylpiperazin, N-(2-Hydroxyethyl)-piperazin, N-(2-Methoxyethyl)-piperazin, N-Propylpiperazin, N-Isopropylpiperazin, N-Butylpiperazin, N-(sek.-Butyl)-piperazin, N-Formylpiperazin, 2-Methylpiperazin, cis- und trans-2,6-Dimethylpiperazin, 2-Phenylpiperazin, 2-(4-Fluorphenyl)-piperazin, 2-(4-Chlorphenyl)-piperazin, 2-(4-Methylphenyl)-piperazin, 2-(4-Methoxyphenyl)-piperazin, 2-(4-Hydroxyphenyl)-piperazin, 2-(2-Thienyl)-piperazin, Pyrrolidin, 3-Amino-pyrrolidin, 3-Aminomethyl-pyrrolidin, 3-Methylaminomethyl-pyrrolidin, 3-Dimethylaminomethyl-pyrrolidin, 3-Ethylaminomethyl-pyrrolidin, 3-Hydroxy-pyrrolidin.

Die als Ausgangsstoffe verwendeten Verbindungen der Formel (V) sind bekannt. Als Beispiele seien genannt: Methyliodid, Methylbromid, Ethyliodid, Ethylbromid, 2-Hydroxyethylchlorid, 3-Hydroxypropylchlorid, n-Propylbromid, Isopropyliodid, n-Butylbromid, sek.-Butyliodid, Isobutylbromid, Ameisensäureessigsäure-anhydrid, Ameisensäureethylester, Ameisensäure, Essigsäureanhydrid, Acetylchlorid.

Die Umsetzung von (II) mit (III) gemäß Methode A wird vorzugsweise in einem Verdünnungsmittel wie Dimethylsulfoxid, N,N-Dimethylformamid, Hexamethyl-phosphorsäuretrisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diaza-bicyclo[2,2,2]-octan (DABCO), 1,8-Diaza-bicyclo[5,4,0]-undec-7-en (DBU) oder überschüssiges Amin (III).

Die Reaktionstemperaturen können in einem größeren Bereich variiert werden. Im allgemeinen arbeitet man zwischen etwa 20 und 200 °C, vorzugsweise zwischen 80 und 180 °C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Carbonsäure (II) 1 bis 15 Mol, vorzugsweise 1 bis 6 Mol des Amins (III) ein.

Freie Aminogruppen können während der Umsetzung durch eine geeignete Aminoschutzgruppe, zum Beispiel der t-Butoxycarbonyl-, Ethoxycarbonyl- oder Acetylgruppe geschützt, und nach Beendigung der Reaktion wieder freigesetzt werden. Eine aromatische Aminogruppe wird über die Reduktion einer Nitrogruppe eingeführt.

Die Umsetzung von (IV) mit (V) wird vorzugsweise in einem Verdünnungsmittel wie Dimethylsulfoxid, Dioxan, N,N-Dimethylformamid, Hexamethyl-phosphorsäure-tris-amid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2,2,2]octan (DABCO) oder 1,8-Diazabicyclo-[5,4,0]undec-7-en (DBU).

Die Reaktionstemperaturen können in einem größeren Bereich variiert werden. Im allgemeinen arbeitet man zwischen etwa 20 und etwa 180° C, vorzugsweise zwischen 40 und 110° C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens gemäß Methode B setzt man auf 1 Mol der Verbindung (IV) 1 bis 4 Mol, vorzugsweise 1 bis 1,5 Mol der Verbindung (V) ein.

Die Umsetzung von (IV) mit (VI) (Methode C) wird vorzugsweise in einem Verdünnungsmittel wie Dioxan, Dimethylsulfoxid, N,N-Dimethylformamid, Methanol, Ethanol, Isopropanol, n-Propanol, Glykolmonomethylether oder auch in Gemischen dieser Verdünnungsmittel durchgeführt.

Die Reaktionstemperaturen können in einem größeren Bereich variiert werden. Im allgemeinen arbeitet man zwischen etwa 20° C und etwa 150° C, vorzugsweise zwischen 50° C und 100° C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens nach Methode C setzt man auf 1 Mol der Verbindung (IV) 1 bis 5 Mol, vorzugsweise 1 bis 2 Mol, der Verbindung (VI) ein.

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel (Methanol, Ethanol, Aceton, Acetonitril). Man kann auch äquivalente Mengen Betain und Säure in Wasser bis zur Lösung erhitzen und anschließend bis zur Trockne eindampfen. Als pharmazeutisch verwendbare Salze sind beispielsweise die Salze von Salzsäure, Schwefelsäure, Essigsäure, Glykolsäure, Milchsäure, Bernsteinsäure, Zitronensäure, Weinsäure, Methansulfonsäure, Galacturonsäure, Gluconsäure, Glutaminsäure oder Asparaginsäure zu verstehen.

Die Alkali- oder Erdalkalisalze werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze der 1,4-Dihydro-4-oxo-1,8-naphthyridin-3-carbonsäuren erhalten.

Außer den in den Beispielen aufgeführten Verbindungen seien als neue Wirkstoffe im einzelnen genannt:
1-Cyclopropyl-6-fluor-1,4-dihydro-7-(3,5-dimethyl-1-piperazinyl)-4-oxo-1,8-naphthyridin-3-carbonsäure,
1-Cycloproypl-6-fluor-1,4-dihydro-7-(4-isopropyl-1-piperazinyl)-4-oxo-1,8-naphthyridin-3-carbonsäure,
7-(4-Butyl-1-piperazinyl)-6-chlor-1-cyclopropyl-1,4-dihydro-4-oxo-1,8-naphthyridin-3-carbonsäure,
1-Cyclopropyl-6-fluor-1,4-dihydro-4-oxo-7-(4-phenacyl-1-piperazinyl)-1,8-naphthyridin-3-carbonsäure,
1-Cyclopropyl-6-fluor-1,4-dihydro-7-[3-methyl-4-(2-oxopropyl)-1-piperazinyl]-4-oxo-1,8-naphthyridin-3-carbonsäure,
6-Chlor-1-cyclopropyl-1,4-dihydro-4-oxo-7-[4-(3-oxobutyl)-1-piperazinyl]-1,8-naphthyridin-3-carbonsäure,
1-Cyclopropyl-6-fluor-7-(4-formyl-1-piperazinyl)-1,4-dihydro-4-oxo-1,8-naphthyridin-3-carbonsäure,
7-(4-Acetyl-1-piperazinyl)-1-cyclopropyl-6-fluor-1,4-dihydro-4-oxo-1,8-naphthyridin-3-carbonsäure,
6-Chlor-1-cyclopropyl-1,4-dihydro-7-(1-piperazinyl)-4-oxo-1,8-naphthyridin-3-carbonsäure,
6-Chlor-1-cyclopropyl-1,4-dihydro-7-(4-methyl-1-piperazinyl)-4-oxo-1,8-naphthyridin-3-carbonsäure,
6-Chlor-1-cyclopropyl-1,4-dihydro-7-(3-methyl-1-piperazinyl)-4-oxo-1,8-naphthyridin-3-carbonsäure,
6-Chlor-1-cyclopropyl-7-(4-ethyl-1-piperazinyl)-1,4-dihydro-4-oxo-1,8-naphthyridin-3-carbonsäure,
6-Chlor-1-cyclopropyl-1,4-dihydro-4-oxo-7-[4-(3-oxopropyl)-1-piperazinyl]-1,8-naphthyridin-3-carbonsäurehydrochlorid,
1-Cyclopropyl-6-fluor-7-[3-(4-fluorphenyl)-1-piperazinyl]-1,4-dihydro-4-oxo-1,8-naphthyridin-3-carbonsäure,
6-Chlor-1-cyclopropyl-1,4-dihydro-4-oxo-7-(3-phenyl-1-piperazinyl)-1,8-naphthyridin-3-carbonsäure,
1-Cyclopropyl-1,4-dihydro-6-nitro-4-oxo-7-(1-piperazinyl)-1,8-naphthyridin-3-carbonsäure,
1-Cyclopropyl-1,4-dihydro-7-(4-methyl-1-piperazinyl)-6-nitro-4-oxo-1,8-naphthyridin-3-carbonsäure,
7-[4-(4-Aminobenzyl)-1-piperazinyl]-6-fluor-1-cyclopropyl-1,4-dihydro-4-oxo-1,8-naphthyridin-3-carbonsäure,

Wertvolle Vorstufen sind Verbindungen der Formel (VII)

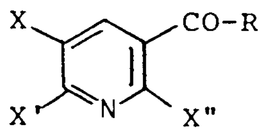

(VII)

in der

X für Halogen oder Nitro steht,

X' und X'' gleich oder verschieden sind und für Halogen, insbesondere Chlor oder Fluor, stehen und

R OH, Halogen, insbesondere Chlor,oder Alkoxycarbonylmethyl mit Methyl oder Ethyl im Alkoxyteil bedeutet.

Die folgenden Beispiele erläutern die Erfindung:

Herstellung der Ausgangsverbindungen

Beispiel A

2,6-Dichlor-3-methyl-5-(3,3-dimethyl-1-triazeno)-pyridin

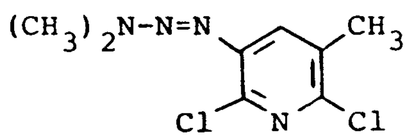

43 g (0,24 mol) 5-Amino-2,6-dichlor-3-methyl-pyridin (Helv. Chim. Acta 59, 190 [1976]) werden langsam mit 285 ml halbkonzentrierter Salzsäure versetzt, auf 0° abgekühlt, dann eine Lösung von 17,2 g (0,25 mol) Natriumnitrit in 70 ml Wasser zugetropft und bei 0° einige Zeit nachgerührt. Diese Diazoniumsalz-Lösung wird bei 0-3° innerhalb 90 Minuten zu einer Lösung aus 150 g Natriumcarbonat in 430 ml Wasser und 70 ml 40 - 50 %iger wäßriger Dimethylamin-Lösung getropft und bei 0° nachgerührt. Man saugt den Niederschlag ab, wäscht gut mit Wasser nach und trocknet im Hochvakuum bei 40° C.
Ausbeute: 49,3 g (88 % der Theorie), Schmelzpunkt: 91-95° C.

Beispiel B

2,6-Dichlor-5-fluor-3-methyl-pyridin

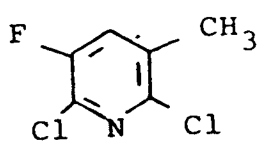

43,9 g (0,19 mol) 2,6-Dichlor-3-methyl-5-(3,3-dimethyl-1-triazeno)-pyridin werden in 80 ml Fluorwasserstoffsäure bei 125-135° C im Autoklaven zersetzt. Nach Destillation erhält man ein Produkt mit einer gaschromatographisch bestimmten Reinheit von 87 %, das außerdem noch 12 % Chlor-Fluor-Austausch-Produkt enthält.
Ausbeute: 19 g, Siedepunkt: 81-95°/18 mbar
Schmelzpunkt: 39-41° C.

Beispiel C

2,6-Dichlor-5-fluor-3-trichlormethyl-pyridin

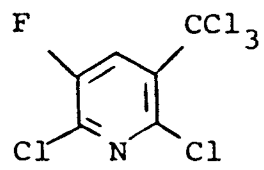

49,4 g (0,27 mol) 2,6-Dichlor-5-fluor-3-methyl-pyridin werden bei 120 °C insgesamt etwa 20 Stunden chloriert, bis NMR-spektroskopisch kein aliphatisches Proton mehr nachweisbar ist. Das Reaktionsgemisch wird in einer Kugelrohrdestillationsapparatur destilliert.
Ausbeute: 61,7 g (80,6 %), Siedepunkt: 130-150 °C (Ofentemperatur) / 0,4 mbar.
Massenspektrum: $^{m}/e$ 281 ($M^+$), 246 (100 %, $M^+$-Cl), 211 (246-Cl), 176 (211-Cl).

Beispiel D

2,6-Dichlor-5-fluor-pyridin-3-carbonsäure

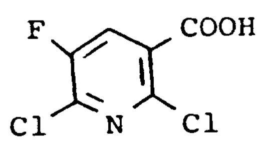

57 g (0,2 mol) 2,6-Dichlor-5-fluor-3-trichlormethylpyridin werden in 53 ml 92 %iger Schwefelsäure gelöst und zunächst 45 Minuten bei 25 °C, danach 3 Stunden bei 100 °C bis zum Abklingen der Chlorwasserstoff-Entwicklung gerührt. Man versetzt mit 24 g 50 %iger Schwefelsäure und erhitzt weitere 6 Stunden auf 100 °C. Dann wird abgekühlt, die Reaktionsmischung auf Eis gegossen, der Niederschlag abgesaugt, mit Wasser gewaschen und getrocknet.
Rohausbeute: 42 g (~ 100 % der Theorie), Schmelzpunkt: 137-149 °C;
nach Umkristallisation aus Wasser: Schmelzpunkt: 154-161 °C.
Massenspektrum: $^{m}/e$ 209 ($M^+$), 192 ($M^+$-OH), 164 (192-CO), 129 (164-Cl), 94 (129-Cl).

Beispiel E

2,6-Dichlor-5-fluor-pyridin-3-carbonylchlorid

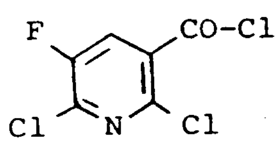

42 g (0,2 mol) 2,6-Dichlor-5-fluor-pyridin-3-carbonsäure werden in einer Mischung aus 43 g Thionylchlorid, 15 ml Dimethylformamid und 640 ml Toluol 6 Stunden unter Rückfluß erhitzt. Die Mischung wird eingeengt und der Rückstand destilliert.
Ausbeute: 33,8 g (74 % der Theorie), Siedepunkt 94-98 °C/1,3 mbar.
Massenspektrum: $^{m}/e$ 227 ($M^+$), 192 (100 %, $M^+$-Cl), 164 (40 %, $M^+$-COCl).

Beispiel F

(2,6-Dichlor-5-fluor-pyridin-3-carbonyl)-essigsäure-ethylester

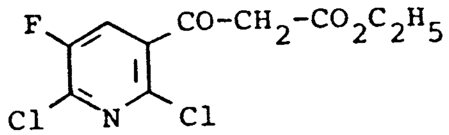

3,7 g (0,15 mol) Magnesiumspäne werden in 9,3 ml Ethanol mit 0,8 g Tetrachlormethan versetzt und nach Beginn der Wasserstoff-Entwicklung ein Gemisch aus 23,9 g (0,15 mol) Malonsäurediethylester, 18,5 ml Ethanol und 58 ml Toluol bei 50-60 ° C tropfenweise zugefügt. Man rührt 1 Stunde bei dieser Temperatur nach, kühlt auf -5 bis -10 ° C und tropft langsam eine Lösung von 31 g (0,14 mol) 2,6-Dichlor-5-fluor-pyridin-3-carbonylchlorid in 14,5 ml Toluol zu. Danach wird 1 Stunde bei 0 ° gerührt, über Nacht auf Raumtemperatur gebracht und noch 2 Stunden auf 40-50 ° C erwärmt. Das Reaktionsgemisch wird unter Eiskühlung mit einem Gemisch aus 60 ml Wasser und 9 ml konzentrierter Schwefelsäure versetzt und die organische Phase abgetrennt. Die wäßrige Phase wird mit Toluol extrahiert und der vereinigte organische Extrakt mit gesättigter Natriumchlorid-Lösung gewaschen, mit Natriumsulfat getrocknet und das Lösungsmittel abgezogen. Man erhält 50,1 g (2,6-Dichlor-5-fluor-pyridin-3-carbonyl)-malonsäurediethylesterals Rohprodukt. Dieses wird nach Zugabe von 50 ml Wasser und 0,1 g 4-Toluolsulfonsäure 10 Stunden unter Rückfluß erhitzt, die Mischung mit Dichlormethan extrahiert, der Extrakt mit Natriumsulfat getrocknet, eingeengt, der Rückstand mit wenig Ether verrührt und das Kristallisat isoliert.
Ausbeute: 14,3 g (34 % der Theorie), Schmelzpunkt: 69-72 ° C.
Massenspektrum: $^{m}/e$ 279 ($M^+$), 244 (60 %, $M^+$-Cl), 216 (74 %, 244-28), 192 (100 %, $C_6HCl_2FNO$), 164, 29.
Nach dem NMR-Spektrum ($CDCl_3$) liegt die Verbindung praktisch vollständig als Enol vor.

Beispiel G

7-Chlor-1-cyclopropyl-6-fluor-1,4-dihydro-4-oxo-1,8-naphthyridin-3-carbonsäure

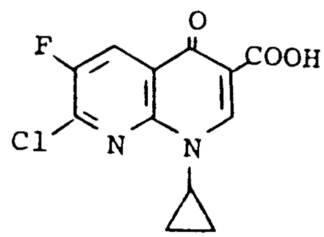

14 g (50 mmol) (2,6-Dichlor-5-fluor-pyridin-3-carbonyl)-essigsäureethylester werden mit 11,1 g (75 mmol) Orthoameisensäuretriethylester in 13 g Essigsäureanhydrid 2 Stunden auf 150-160 ° C erhitzt. Man engt im Vakuum ein und erhält 15,6 g 2-(2,6-Dichlor-5-fluor-pyridin-3-carbonyl)-3-ethoxyacrylsäureethylester als öligen Rückstand.

15,5 g (46 mmol) dieser Zwischenstufe werden in 35 ml Ethanol unter Eiskühlung tropfenweise mit 3 g Cyclopropylamin versetzt und 1 Stunde bei 20 ° C gerührt. Das ausgefallene Produkt wird abgesaugt, mit Methanol gewaschen und getrocknet. Man erhält 13,3 g 2-(2,6-Dichlor-5-fluor-pyridin-3-carbonyl)-3-cyclopropylaminoacrylsäureethylester vom Schmelzpunkt 130-133 ° C (aus Ethanol).

12,5 g (36 mmol) 2-(2,6-Dichlor-5-fluor-pyridin-3-carbonyl)-3-cyclopropylamino-acrylsäureethylester werden in 75 ml Dimethylformamid mit 6,5 g Kaliumcarbonat 1 Stunde auf 100 ° C erhitzt. Das Reaktionsgemisch wird auf Eiswasser gegossen und das ausgefallene Produkt abgesaugt, mit Wasser und Methanol gewaschen und getrocknet. Es werden 10,5 g (94 % der Theorie) 7-Chlor-1-cyclopropyl-6-fluor-1,4-dihydro-4-oxo-1,8-naphthyridin-3-carbonsäureethylester vom Schmelzpunkt 176-180 ° C erhalten.

10,5 g (34 mmol) dieses Esters werden in einem Gemisch aus 100 ml Essigsäure, 70 ml Wasser und 10 ml konzentrierter Schwefelsäure 2 Stunden auf 150 ° C erhitzt. Die Suspension wird in 300 ml Eiswasser gegossen, der Niederschlag abgesaugt, mit Wasser und Methanol gewaschen und im Vakuum getrocknet.
Ausbeute: 7,85 g (82 % der Theorie) 7-Chlor-1-cyclopropyl-6-fluor-1,4-dihydro-4-oxo-1,8-naphthyridin-3-carbonsäure vom Schmelzpunkt 230-233 ° C.

Beispiel H

6,7-Dichlor-1-cyclopropyl-1,4-dihydro-4-oxo-1,8-naphthyridin-3-carbonsäure

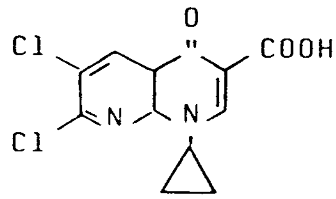

Ausgehend von 2,5,6-Trichlorpyridin-3-carbonsäurechlorid [Helv. Chim. Acta 59, 222 (1976)] wird analog Beispiel F (2,5,6-Trichlorpyridin-3-carbonyl)-essigsäureethylester (Schmelzpunkt: 69-71°; liegt nach dem $^1$H-NMR-Spektrum in Deuterochloroform zu 50 % als Enol vor) hergestellt. Dieser wird anschließend analog Beispiel G über 6,7-Dichlor-1-cyclopropyl-1,4-dihydro-4-oxo-1,8-naphthyridin-3-carbonsäure-ethylester (Schmelzpunkt: 176-178°) zur 6,7-Dichlor-1-cyclopropyl-1,4-dihydro-4-oxo-1,8-naphthyridin-3-carbonsäure umgesetzt, die nach Umkristallisation aus Dimethylformamid bei 243-245° unter Zersetzung schmilzt.

Beispiel 1

1-Cyclopropyl-6-fluor-1,4-dihydro-7-(3-methyl-1-piperazinyl)-4-oxo-1,8-naphthyridin-3-carbonsäure

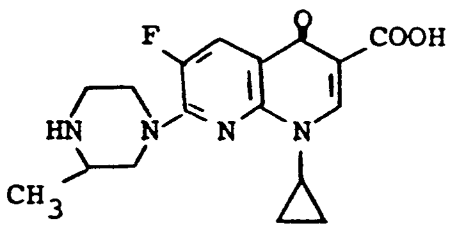

1,4 g (4 mmol) 7-Chlor-1-cyclopropyl-6-fluor-1,4-dihydro-4-oxo-1,8-naphthyridin-3-carbonsäure werden in 8 ml Dimethylsulfoxid mit 860 mg (10 mmol) wasserfreiem 2-Methylpiperazin 15 Minuten auf 110°C erhitzt. Das Lösungsmittel wird im Vakuum abgedampft, der Rückstand mit 5 ml Wasser aufgekocht (pH 7), der Niederschlag abgesaugt, mit Wasser gewaschen und mit Methanol aufgekocht.
Ausbeute: 0,9 g (65 % der Theorie), Schmelzpunkt: 243-247°C (unter Zersetzung)aus Glykolmonomethylether umkristallisiert

Beispiel 2

1-Cyclopropyl-7-(4-ethyl-1-piperazinyl)-6-fluor-1,4-dihydro-4-oxo-1,8-naphthyridin-3-carbonsäure-hydrochlorid

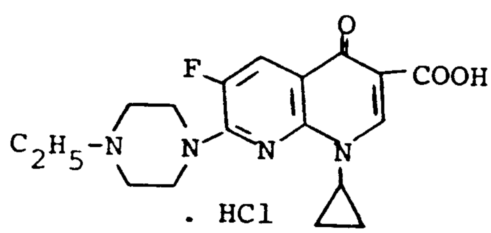

Analog Beispiel 3 wird mit N-Ethyl-piperazin 30 Minuten bei 100°C umgesetzt und das Reaktionsprodukt anschließend in das Hydrochlorid überführt.
Ausbeute: 1,05 g (66 % der Theorie), Schmelzpunkt: > 300°C (unter Zersetzung).

Beispiel 3

1-Cyclopropyl-6-fluor-1,4-dihydro-7-[4-(2-hydroxyethyl)-1-piperazinyl]-4-oxo-1,8-naphthyridin-3-carbonsäure

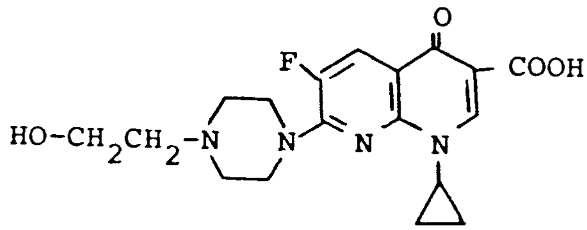

Analog Beispiel 1 wird mit N-(2-Hydroxyethyl)-piperazin 30 Minuten bei 100 ° C umgesetzt und das Reaktionsprodukt aus Glykolmonomethylether umkristallisiert.
Ausbeute: 0,9 g (60 % der Theorie), Schmelzpunkt: 241-245 ° C (unter Zersetzung).

Beispiel 4

1-Cyclopropyl-6-fluor-1,4-dihydro-4-oxo-7-(3-phenyl-1-piperazinyl)-1,8-naphthyridin-3-carbonsäure

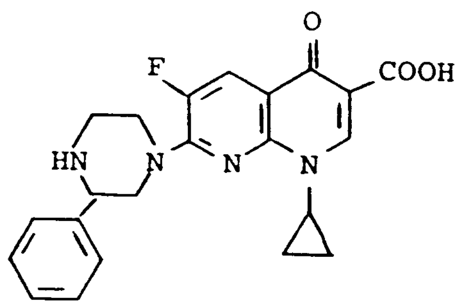

Analog Beispiel 1 werden 810 mg (5 mmol) 2-Phenylpiperazin in Gegenwart von 1,8 g (8 mmol) 1,4-Diaza-bicyclo[2,2,2]octan (DABCO) 30 Minuten bei 100 ° C umgesetzt.
Ausbeute: 0,85 g (42 % der Theorie), Schmelzpunkt: 280-283 ° C (unter Zersetzung).

Beispiel 5

1-Cyclopropyl-6-fluor-1,4-dihydro-4-oxo-7-[4-(2-oxopropyl)-1-piperazinyl]-1,8-naphthyridin-3-carbonsäure-Hydrochlorid

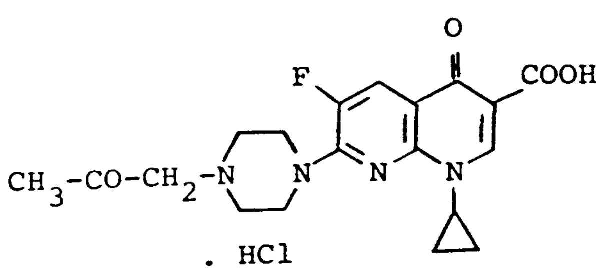

1,65 g (5 mmol) 1-Cyclopropyl-6-fluor-1,4-dihydro-4-oxo-7-(1-piperazinyl)-1,8-naphthyridin-3-carbonsäure werden in 25 ml Dimethylformamid mit 0,7 g (7,6 mmol) Chloraceton und 1,05 g Triethylamin versetzt und 3 Stunden auf 80 ° C erhitzt. Die Suspension wird im Vakuum eingeengt, der Rückstand mit 10 ml Wasser verrührt, abgesaugt und getrocknet. Das Produkt wird in 15 ml verdünnter Salzsäure (1:1) erhitzt, mit Ethanol ausgefällt, abgesaugt und getrocknet.
Ausbeute: 1,5 g (71 % der Theorie), Schmelzpunkt: > 300 ° C (unter Zersetzung).

Beispiel 6

1-Cyclopropyl-6-fluor-1,4-dihydro-4-oxo-7-[4-(3-oxo-butyl)-1-piperazinyl]-1,8-naphthyridin-3-carbonsäure-Hydrochlorid

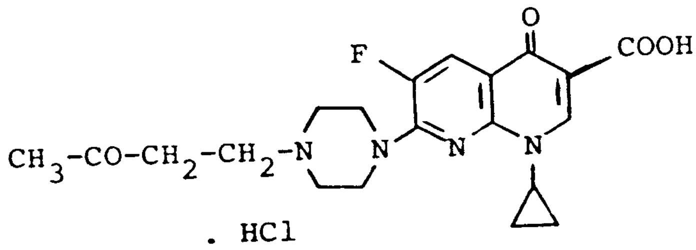

1,66 g (5 mmol) der Verbindung aus Beispiel 1 und 1,95 g (28 mmol) Methylvinylketon werden in 25 ml Ethanol 7 Stunden unter Rückfluß erhitzt, der erhaltene Niederschlag mit verdünnter Salzsäure (1:1) gelöst und mit Ethanol ausgefällt.
Ausbeute: 1,1 g (55 % der Theorie), Schmelzpunkt: > 300 ° C (unter Zersetzung).

Beispiel 7

6-Chlor-1-cyclopropyl-1,4-dihydro-7-(4-methyl-1-piperazinyl)-4-oxo-1,8-naphthyridin-3-carbonsäure-Hydrochlorid

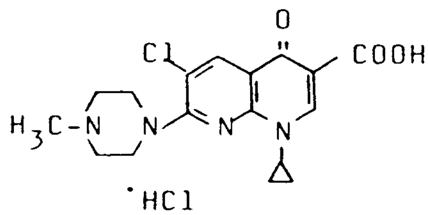

Man verfährt analog Beispiel 3, wobei man von 6,7-Dichlor-1-cyclopropyl-1,4-dihydro-4-oxo-1,8-naphthyridin-3-carbonsäure und N-Methyl-piperazin als Ausgangsverbindungen ausgeht.
Ausbeute: 66 %, Schmelzpunkt: 304 - 308 ° (unter Zersetzung).

Beispiel für eine erfindungsgemäße Tablette

Jede Tablette enthält:

| | |
|---|---|
| Verbindung des Beispiels 1 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |

Die Lackhülle enthält:

| | |
|---|---|
| Poly-(O-hydroxypropyl-O-methyl)-cellulose 15 cp<br>Macrogol 4000 rec. INN (Polyethylenglykol DAB)<br>Titan-(IV)-oxid | 6,0 mg<br>2,0 mg<br>2,0 mg |
| | 10,0 mg |

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium, ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die erfindungsgemäßen Verbindungen verhindert, gebessert und/oder geheilt werden können, seien beispielsweise genannt:

Otitis; Pharyngitis; Pneumonie; Peritonitis; Pyelonephritis; Cystitis; Endocarditis; Systeminfektionen; Bronchitis; Arthritis; lokale Infektionen; septische Erkrankungen.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch Pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z. B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gramnegative oder gram-positive Bakterien verhindert, gebessert und/oder geheilt werden und dadurch eine Förderung des Wachstums und eine Verbesserung der Verwertung des Futters erreicht werden.

In der nachstehenden Tabelle sind die MHK-Werte einiger der erfindungsgemäßen Verbindungen angegeben.

Als Vergleich wurden die entsprechenden MHK-Werte der aus der Europäischen Patentanmeldung 9425, den Japanischen Patentanmeldungen 81/45473 [C.A. 95, 115 597 (1981)], 81/46811 [C.A. 95, 121 142 (1981)], aus J. Med. Chem. 27, 292 (1984) oder aus J. Heterocycl. Chem. 21, 673 (1984) bekannten 1-Ethyl-6-fluor-1,4-dihydro-4-oxo-7-(1-piperazinyl)-1,8-naphthyridin-3-carbonsäure (AT 2266, Enoxacin) angegeben, wobei sich zeigt, daß die erfindungsgemäßen Verbindungen der bekannten Verbindung überlegen sind.

Tabelle 1: MHK-Werte (mcg/ml)

| Stamm | Beispiel 1 | Enoxacin |
|---|---|---|
| E.coli 4418 | 0.03 | 0.25 |
| E.coli Neum. | ≤ 0.015 | 0.06 |
| E.Coli 455/7 | 8 | 16 |
| Klebsiella 63 | ≤ 0.015 | 0,5 |
| Klebsiella 6179 | 0.125 | 2 |
| Proteus mir. 8175 | 0.25 | 0.25 |
| Proteus vulg. 1017 | 0.125 | 0.125 |
| Proteus morg. 11006 | 0.03 | 0.125 |
| Providencia 12012 | 0.125 | 0.25 |
| Serratia 16040 | 8 | 32 |
| Staphyloc. 1756 | 0.5 | 1 |
| Staphyloc. 133 | 0.5 | 1 |
| Pseudomonas W. | 1 | 2 |

Agardilutionstest / Isosensitestmedium

## Patentansprüche

**Patentansprüche für folgende Vertragsstaaten : BE, CH, DE, FR, GB, IT, LI, NL, SE**

**1.** 1-Cyclopropyl-1,4-dihydro-4-oxo-1,8-naphthyridin-3-carbonsäuren der Formel (I)

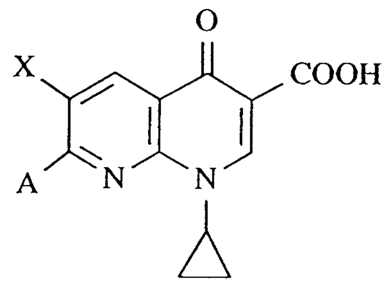

(I)

in welcher
X für Halogen und
A für

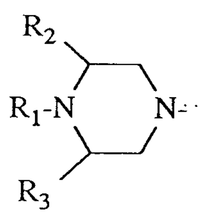

oder

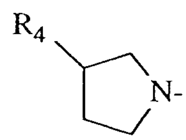

stehen
worin
$R_1$ für verzweigtes oder geradkettiges Alkyl mit 1 - 4 Kohlenstoffatomen steht, das durch eine Hydroxy- oder Ethoxygruppe substituiert sein kann, einen gegebenenfalls durch Hydroxy, Methoxy, Chlor oder Fluor substituierten Phenacylrest, Oxopropyl, 2-Oxobutyl oder für den Rest

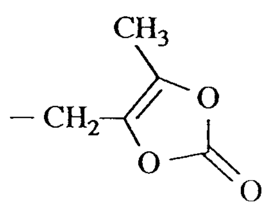

steht,
$R_2$ für Wasserstoff oder Methyl oder gegebenenfalls durch Chlor, Fluor, Methyl, Hydroxy oder Methoxy substituiertes Phenyl oder Thienyl,
$R_3$ für Wasserstoff oder Methyl oder
$R_4$ für Hydroxymethyl steht,

sowie deren pharmazeutisch verwendbare Hydrate, Säureadditionssalze, Alkali-, Erdalkali-, Silber- und Guanidiniumsalzen sowie ihren Methyl-, Ethyl-, Pivaloyloxymethyl-, Pivaloyloxyethyl oder deren (5-Methyl-2-oxo-1,3-dioxol-4-yl-methyl)ester, wobei $R_1$ nicht Methyl sein kann wenn X Fluor bedeutet.

**2.** 1-Cyclopropyl-1,4-dihydro-4-oxo-1,8-naphthyridin-3-carbonsäuren gemäß Anspruch 1,

in welchen

X für Fluor oder Chlor und
A für

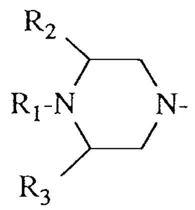

oder

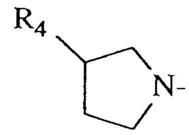

stehen
worin

$R_1$ für verzweigtes oder geradkettiges Alkyl mit 1 -4 Kohlenstoffatomen steht, das durch eine Hydroxy- oder Ethoxygruppe substituiert ist, einen durch Hydroxy, Methoxy, Chlor oder Fluor substituierten Phenacylrest, 2-Oxopropyl, 2-Oxobutyl
oder für den Rest

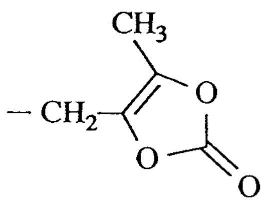

steht,

$R_2$ für Wasserstoff oder Methyl oder gegebenenfalls durch Chlor, Fluor, Methyl, Hydroxy oder Methoxy substituiertes Phenyl oder Thienyl,
$R_3$ für Wasserstoff oder Methyl und
$R_4$ für Hydroxymethyl steht,

sowie deren pharmazeutisch verwendbare Hydrate, Säureadditionssalze, Alkali-, Erdalkali-, Silber- und Guanidiniumsalzen sowie ihren Methyl-, Ethyl-, Pivaloyloxymethyl-, Pivaloyloxyethyl oder deren (5-Methyl-2-oxo-1,3-dioxol-4-yl-methyl)ester, wobei $R_1$ nicht Methyl sein kann wenn X Fluor bedeutet.

**3.** 1-Cyclopropyl-1,4-dihydro-4-oxo-1,8-naphthyridin-3-carbonsäuren gemäß Anspruch 1,

in welcher
X für Fluor oder Chlor, und
A für

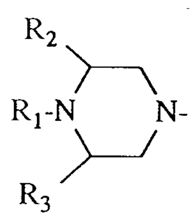

steht,
worin

$R_1$ für verzweigtes oder geradkettiges Alkyl mit 1 - 4 Kohlenstoffatomen steht, das durch eine Hydroxy- oder Ethoxygruppe substituiert ist, einen gegebenenfalls durch Hydroxy, Methoxy, Chlor oder Fluor substituierten Phenacylrest, 2-Oxopropyl, 2-Oxobutyl
oder für den Rest

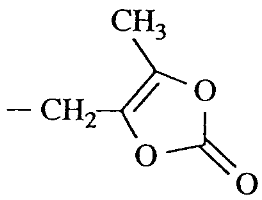

steht,

$R_2$ für Wasserstoff oder Methyl oder gegebenenfalls durch Chlor, Fluor, Methyl, Hydroxy oder Methoxy substituiertes Phenyl oder Thienyl,

$R_3$ für Wasserstoff oder Methyl steht,

sowie deren pharmazeutisch verwendbare Hydrate, Säureadditionssalze, Alkali-, Erdalkali-, Silber- und Guanidiniumsalzen sowie ihren Methyl-, Ethyl-, Pivaloyloxymethyl-, Pivaloyloxyethyl oder deren (5-Methyl-2-oxo-1,3-dioxol-4-yl-methyl)ester, wobei $R_1$ nicht Methyl sein kann wenn X Fluor bedeutet.

**4.** 1-Cyclopropyl-6-fluor-1,4-dihydro-7-(3-methyl-1-piperazinyl)-4-oxo-1,8-naphthyridin-3-carbonsäure.

**5.** 1-Cyclopropyl-6-fluor-1,4-dihydro-7-(4(2-hydroxyethyl)-1-piperazinyl)-4-oxo-1,8-naphthyridin-3-carbonsäure.

**6.** Verfahren zur Herstellung von 1-Cyclopropyl-1,4-dihydro-4-oxo-1,8-naphthyridin-3-carbonsäuren gemäß Anspruch 1, dadurch gekennzeichnet daß man 1-Cyclopropyl-7-halogen-1,4-dihydro-4-oxo-1,8-naphthyridin-3-carbonsäuren der Formel (II)

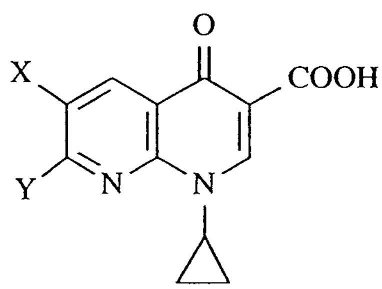

(II)

in welcher X die oben angegebene Bedeutung hat und Y für Halogen, bevorzugt Chlor oder Fluor steht, mit Aminen der Formel (III)

A - H (III)

in welcher A die oben angegebene Bedeutung hat, gegebenenfalls in Gegenwart von Säurebindern umsetzt und gegebenenfalls in die Salze, Ester und andere Prodrug-Formen überführt.

**7.** Arzneimittel, enthaltend 1-Cyclopropyl-1,4-dihydro-4-oxo-1,8-naphthyridin-3-carbonsäuren gemäß Anspruch 1.

**8.** Verwendung von 1-Cyclopropyl-1,4-dihydro-4-oxo-1,8-naphthyridin-3-carbonsäuren gemäß Anspruch 1 zur Herstellung von Arzneimitteln.

**Patentansprüche für folgenden Verstragsstaaten : AT**

**1.** Verfahren zur Herstellung von 1-Cyclopropyl-1,4-dihydro-4-oxo-1,8-naphthyridin-3-carbonsäuren der Formel (I)

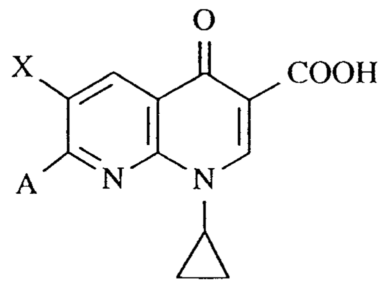

(I)

in welcher

X für Halogen und

A für

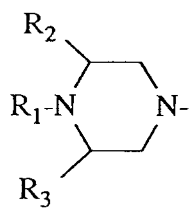

oder

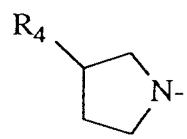

stehen

worin

$R_1$ für verzweigtes oder geradkettiges Alkyl mit 1 - 4 Kohlenstoffatomen steht, das durch eine Hydroxy- oder Ethoxygruppe substituiert sein kann, einen gegebenenfalls durchHydroxy, Methoxy, Chlor oder Fluor substituierten Phenacylrest, 2-Oxopropyl, 2-Oxobutyl oder für den Rest

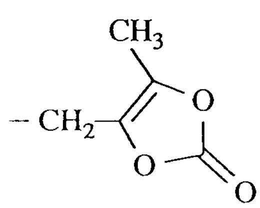

steht,

$R_2$ für Wasserstoff oder Methyl oder gegebenenfalls durch Chlor, Fluor, Methyl, Hydroxy oder Methoxy substituiertes Phenyl oder Thienyl,

$R_3$ für Wasserstoff oder Methyl und

$R_4$ für Hydroxymethyl steht,

sowie deren pharmazeutisch verwendbare Hydrate, Säureadditionssalze, Alkali-, Erdalkali-, Silber- und Guanidiniumsalzen sowie ihren Methyl-, Ethyl-, Pivaloyloxymethyl-, Pivaloyloxyethyl oder deren (5-Methyl-2-oxo-1,3-dioxol-4-yl-methyl)ester, wobei $R_1$ nicht Methyl sein kann wenn X Fluor bedeutet, dadurch gekennzeichnet, daß man 1-Cyclopropyl-7-halogen-1,4-dihydro-4-oxo-1,8-naphthyridin-3-carbonsäuren der Formel (II)

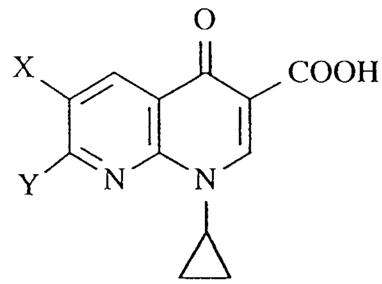

(II)

in welcher X die oben angegebene Bedeutung hat und Y für Halogen, bevorzugt Chlor oder Fluor steht, mit Aminen der Formel (III)

A - H (III),

in welcher A die oben angegebene Bedeutung hat,
gegebenenfalls in Gegenwart von Säurebindern umsetzt und gegebenenfalls in die Salze, Ester und andere Prodrug-Formen überführt.

**2.** Verfahren zur Herstellung von 1-Cyclopropyl-1,4-dihydro-4-oxo-1,8-naphthyridin-3-carbonsäuren gemäß Anspruch 1,

in welchen
X für Fluor oder Chlor und
A für

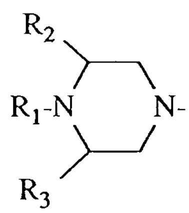

oder

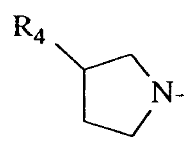

stehen
worin
$R_1$ für verzweigtes oder geradkettiges Alkyl mit 1 - 4 Kohlenstoffatomen steht, das durch eine Hydroxy- oder Ethoxygruppe substituiert ist, einen durch Hydroxy, Methoxy, Chlor oder Fluor substituierten Phenacylrest 2-Oxopropyl, 2-Oxobutyl oder für den Rest

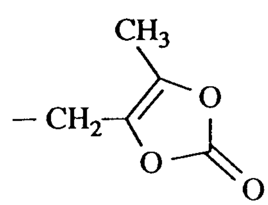

steht,
$R_2$ für Wasserstoff oder Methyl oder gegebenenfalls durch Chlor, Fluor, Methyl, Hydroxy oder Methoxy substituiertes Phenyl oder Thienyl,
$R_3$ für Wasserstoff oder Methyl und
$R_4$ für Hydroxymethyl steht,

sowie deren pharmazeutisch verwendbare Hydrate, Säureadditionssalze, Alkali-, Erdalkali-, Silber- und Guanidiniumsalzen sowie ihren Methyl-, Ethyl-, Pivaloyloxymethyl-, Pivaloyloxyethyl oder deren (5-Methyl-2-oxo-1,3-dioxol-4-yl-methyl)ester, wobei $R_1$ nicht Methyl sein kann wenn X Fluor bedeutet, dadurch gekennzeichnet, daß man 1-Cyclopropyl-7-halogen-1,4-dihydro-oxo-1,8-naphthyridin-3-carbonsäuren der Formel (II)

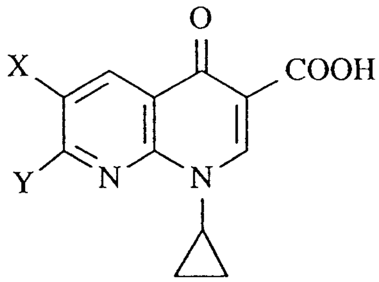

(II)

in welcher X die oben angegebene Bedeutung hat und Y für Halogen, bevorzugt Chlor oder Fluor steht, mit Aminen der Formel (III)

A - H (III),

in welcher A die oben angegebene Bedeutung hat,
gegebenenfalls in Gegenwart von Säurebindern umsetzt und gegebenenfalls in die Salze, Ester und andere Prodrug-Formen überführt.

## Claims

**Claims for the following Contracting States : BE, CH, DE, FR, GB, IT, LI, NL, SE**

**1.** 1-Cyclopropyl-1,4-dihydro-4-oxo-1,8-naphthyridine-3-carboxylic acids of the formula (I)

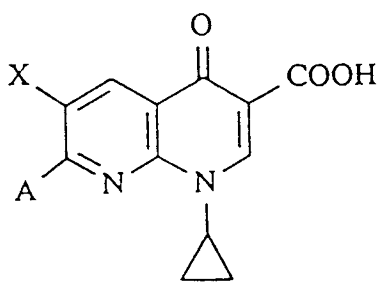

(I)

in which

X represents halogen and
A represents

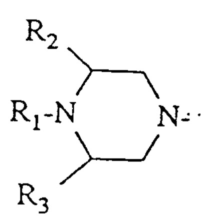

or

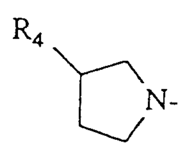

wherein

$R_1$ represents branched or straight-chain alkyl with 1 to 4 carbon atoms, which can optionally be substituted by a hydroxyl or ethoxy group, a phenacyl radical which is optionally substituted by hydroxyl, methoxy, chlorine or fluorine, oxopropyl or 2-oxobutyl or represents the radical

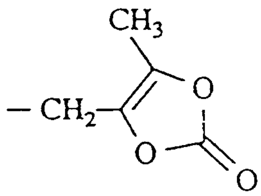

R2 represents hydrogen or methyl, or phenyl or thienyl which is optionally substituted by chlorine, fluorine, methyl, hydroxyl or methoxy,

R3 represents hydrogen or methyl and

R4 represents hydroxymethyl,

and pharmaceutically usable hydrates, acid addition salts and alkali metal, alkaline earth metal, silver and guanidinium salts thereof, and their methyl, ethyl, pivaloyloxymethyl, pivaloyloxyethyl and (5-methyl-2-oxo-1,3-dioxol-4-yl-methyl)esters, and $R_1$ may not be methyl when X denotes fluorine.

**2.** 1-Cyclopropyl-1,4-dihydro-4-oxo-1,8-naphthyridine-3-carboxylic acids according to Claim 1,

in which

X represents fluorine or chlorine and

A represents

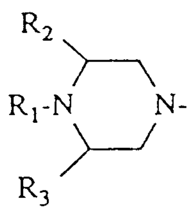

or

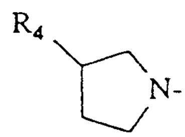

wherein

$R_1$ represents branched or straight-chain alkyl with 1 to 4 carbon atoms, which is substituted by a hydroxyl or ethoxy group, a phenacyl radical which is substituted by hydroxyl, methoxy, chlorine or fluorine, 2-oxopropyl or 2-oxobutyl or represents the radical

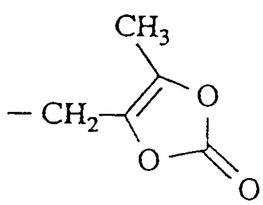

$R_2$ represents hydrogen or methyl, or phenyl or thienyl which is optionally substituted by chlorine, fluorine, methyl, hydroxyl or methoxy,

$R_3$ represents hydrogen or methyl and

$R_4$ represents hydroxymethyl,

and pharmaceutically usable hydrates, acid addition salts and alkali metal, alkaline earth metal, silver and guanidinium salts thereof, and their methyl, ethyl, pivaloyloxymethyl, pivaloyloxyethyl and (5-methyl-2-oxo-1,3-dioxol-4-yl-methyl) esters, and $R_1$ may not be methyl when X denotes fluorine.

**3.** 1-Cyclopropyl-1,4-dihydro-4-oxo-1,8-naphthyridine-3-carboxylic acids according to Claim 1,

in which

X represents fluorine or chlorine and

A represents

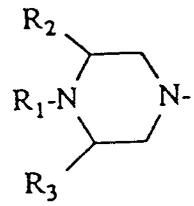

wherein

$R_1$ represents branched or straight-chain alkyl with 1-4 carbon atoms, which is substituted by a hydroxyl or ethoxy group, a phenacyl radical which is optionally substituted by hydroxyl, methoxy, chlorine or fluorine, 2-oxopropyl or 2-oxobutyl or represents the radical

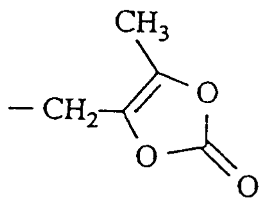

$R_2$ represents hydrogen or methyl, or phenyl or thienyl which is optionally substituted by chlorine, fluorine, methyl, hydroxyl or methoxy

and pharmaceutically usable hydrates, and

$R_3$ represents hydrogen or methyl acid addition salts and alkali metal, alkaline earth metal, silver and guanidinium salts thereof, and their methyl, ethyl, pivaloyloxymethyl, pivaloyloxyethyl and (5-methyl-2-oxo-1,3-dioxol-4-yl-methyl) esters, and $R_1$ may not be methyl when X denotes fluorine.

**4.** 1-Cyclopropyl-6-fluoro-1,4-dihydro-7-(3-methyl-1-piperazinyl)-4-oxo-1,8-naphthyridine-3-carboxylic acid.

**5.** 1-Cyclopropyl-6-fluoro-1,4-dihydro-7-(4(2-hydroxyethyl)-1-piperazinyl)-4-oxo-1,8-naphthyridine-3-carboxylic acid.

**6.** Process for the preparation of 1-cyclopropyl-1,4-dihydro-4-oxo-1,8-naphthyridine-3-carboxylic acids according to Claim 1, characterised in that 1-cyclopropyl-7-halogeno-1,4-dihydro-4-oxo-1,8-naphthyri dine-3-carboxylic acids of the formula (II)

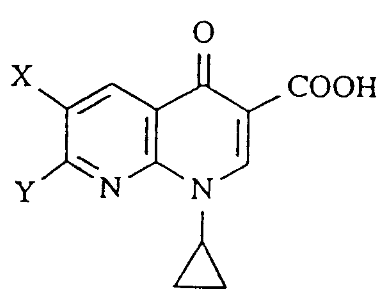

(II)

in which X has the abovementioned meaning and

Y represents halogen, preferably chlorine or fluorine, are reacted with amines of the formula (III)

A - H (III)

in which A has the abovementioned meaning, if appropriate in the presence of acid-binders and, if appropriate, the products are converted into the salts, esters and other prodrug forms.

**7.** Medicaments containing 1-cyclopropyl-1,4-dihydro-4-oxo-1,8-naphthyridine-3-carboxylic acids according to Claim 1.

**8.** Use of 1-cyclopropyl-1,4-dihydro-4-oxo-1,8-naphthyridine-3-carboxylic acids according to Claim 1 for the preparation of medicaments.

**Claims for the following Contracting State : AT**

**1.** Process for the preparation 1-cyclopropyl-1,4-dihydro-4-oxo-1,8-naphthyridine-3-carboxylic acids of the formula (I)

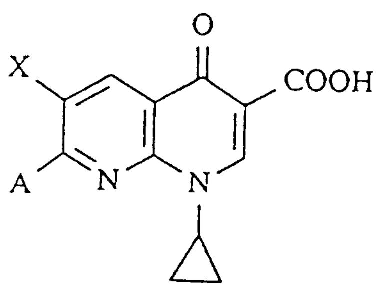

(I)

in which
X represents halogen and
A represents

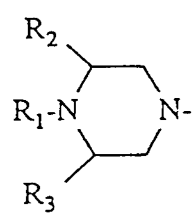

or

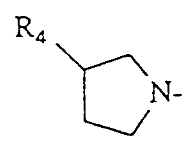

wherein
$R_1$ represents branched or straight-chain alkyl with 1 to 4 carbon atoms, which can optionally be substituted by a hydroxyl or ethoxy group, a phenacyl radical which is optionally substituted by hydroxyl, methoxy, chlorine or fluorine, 2-oxopropyl or 2-oxobutyl or represents the radical

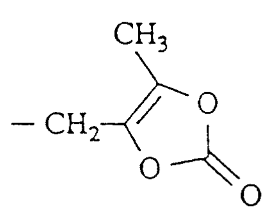

$R_2$ represents hydrogen or methyl, or phenyl or thienyl which is optionally substituted by chlorine, fluorine, methyl, hydroxyl or methoxy,
$R_3$ represents hydrogen or methyl and
$R_4$ represents hydroxymethyl,
and pharmaceutically usable hydrates, acid addition salts and alkali metal, alkaline earth metal, silver

and guanidinium salts thereof, and their methyl, ethyl, pivaloyloxymethyl, pivaloyloxyethyl and (5-methyl-2-oxo-1,3-dioxol-4-yl-methyl) esters, and $R_1$ may not be methyl when X denotes fluorine, characterised in that 1-cyclopropyl-7-halogeno-1,4-dihydro-4-oxo-1,8-naphthyridine-3-carborylic acids of the formula (II)

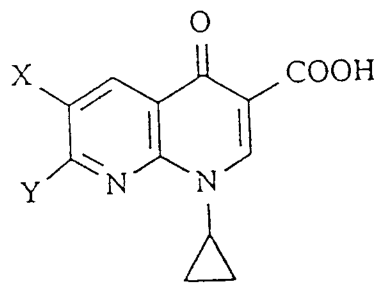

(II)

in which X has the abovementioned meaning and Y represents halogen, preferably chlorine or fluorine, are reacted with amines of the formula (III)

A - H (III)

in which A has the abovementioned meaning,
if appropriate in the presence of acid-binders and, if appropriate, the products are converted into the salts, esters and other prodrug forms.

**2.** Process for the preparation of 1-cyclopropyl-1,4-dihydro-4-oxo-1,8-naphthyridine-3-carboxylic acids according to Claim 1,
in which

X represents fluorine or chlorine and
A represents

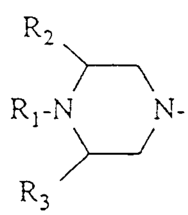

or

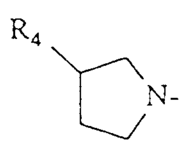

wherein

$R_1$ represents branched or straight-chain alkyl with 1 to 4 carbon atoms, which is substituted by a hydroxyl or ethoxy group, a phenacyl radical which is substituted by hydroxyl, methoxy, chlorine or fluorine, 2-oxopropyl or 2-oxobutyl or represents the radical

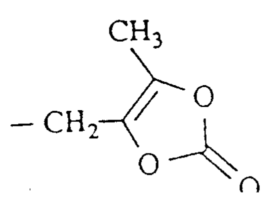

$R_2$ represents hydrogen or methyl, or phenyl or thienyl which is optionally substituted by chlorine, fluorine, methyl, hydroxyl or methoxy,
$R_3$ represents hydrogen or methyl and
$R_4$ represents hydroxymethyl,

and pharmaceutically usable hydrates, acid addition salts and alkali metal, alkaline earth metal, silver

and guanidinium salts thereof, and their methyl, ethyl, pivaloyloxymethyl, pivaloyloxyethyl and (5-methyl-2-oxo-1,3-dioxol-4-yl-methyl) esters, and $R_1$ may not be methyl when X denotes fluorine, characterised in that 1-cyclopropyl-7-halogeno-1,4-dihydro-4-oxo-1,8-naphthyridine-3-carboxylic acids of the formula (II)

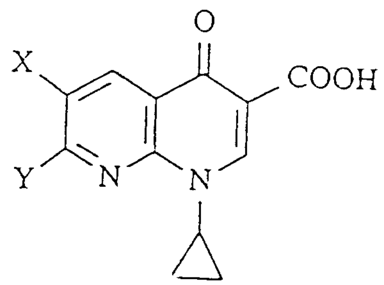

(II)

in which X has the abovementioned meaning and Y represents halogen, preferably chlorine or fluorine, are reacted with amines of the formula (III)

A - H (III)

in which A has the abovementioned meaning,
if appropriate in the presence of acid-binders and, if appropriate, the products are converted into the salts, esters and other prodrug forms.

## Revendications

### Revendications pour les Etats contractants suivants : BE, CH, DE, FR, GB, IT, LI, NL, SE

**1.** Acides 1-cyclopropyl-1,4-dihydro-4-oxo-1,8-naphtyridine-3-carboxyliques de formule I :

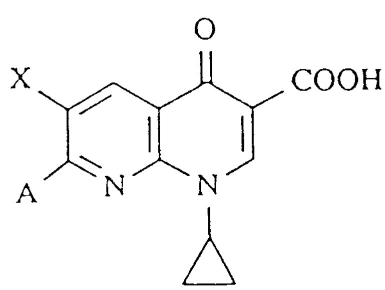

(I)

dans laquelle
X représente un halogène et
A représente

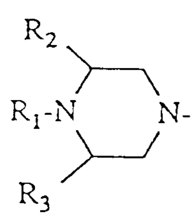

ou

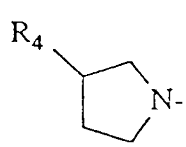

dans lesquels
$R_1$ représente un groupe alkyle à chaîne droite ou ramifiée en $C_1$-$C_4$ qui peut être substitué par un groupe hydroxy ou éthoxy, un groupe phénacyle éventuellement substitué par des groupes hydroxy,

méthoxy, le chlore ou le fluor, un groupe oxopropyle, 2-oxo-butyle ou le groupe

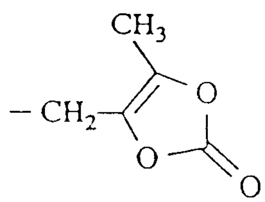

$R_2$ représente l'hydrogène ou un groupe méthyle ou un groupe phényle ou thiényle éventuellement substitué par le chlore, le fluor, des groupes méthyle, hydroxy ou méthoxy,
$R_3$ représente l'hydrogène ou un groupe méthyle, ou bien
$R_4$ représente un groupe hydroxyméthyle,
et leurs hydrates, sels formés par addition avec des acides, sels alcalins, alcalino-terreux, d'argent et de guanidinium acceptables pour l'usage pharmaceutique, et leurs esters méthyliques, éthyliques, pivaloyloxyméthyliques, pivaloyloxyétyliques ou 5-méthyl-2-oxo-1,3-dioxole-4-yl-méthyliques,
sous réserve que $R_1$ ne peut représenter un groupe méthyle lorsque X représente le fluor.

**2.** Acides 1-cyclopropyl-1,4-dihydro-4-oxo-1,8-naphtyridine-3-carboxyliques selon la revendication 1, dans lesquels
X représente le fluor ou le chlore et
A représente

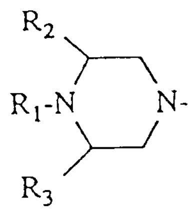

ou

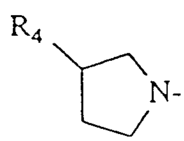

dans lesquels
$R_1$ représente un groupe alkyle à chaîne droite ou ramifiée en $C_1$-$C_4$ qui est substitué par un groupe hydroxy ou éthoxy, un groupe phénacyle substitué par un groupe hydroxy, méthoxy, le chlore ou le fluor, un groupe 2-oxopropyle, 2-oxobutyle, ou le groupe

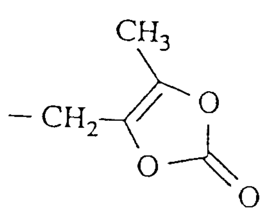

$R_2$ représente l'hydrogène ou un groupe méthyle, ou un groupe phényle ou thiényle éventuellement substitué par le chlore, le fluor, des groupes méthyle, hydroxy ou méthoxy,
$R_3$ représente l'hydrogène ou un groupe méthyle et
$R_4$ représente un groupe hydroxyméthyle,
et leurs hydrates, sels formés par addition avec des acides, sels alcalins, alcalino-terreux, d'argent et de guanidinium acceptables pour l'usage pharmaceutique, ainsi que leurs esters méthyliques, éthyliques, pivaloyloxyméthyliques, pivaloyloxyéthyliques ou 5-méthyl-2-oxo-1,3-dioxole-4-yl-méthyliques,
sous réserve que $R_1$ ne peut représenter un groupe méthyle lorsque X représente le fluor.

**3.** Acides 1-cyclopropyl-1,4-dihydro-4-oxo-1,8-naphtyridine-3-carboxyliques selon la revendication 1, dans lesquels
X représente le fluor ou le chlore et

A représente

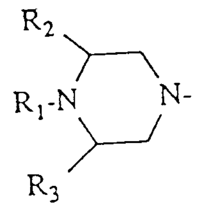

dans lequel

$R_1$ représente un groupe alkyle à chaîne droite ou ramifiée en $C_1$-$C_4$ qui est substitué par un groupe hydroxy ou éthoxy, un groupe phénacyle éventuellement substitué par des groupes hydroxy, méthoxy, le chlore ou le fluor, un groupe 2-oxopropyle, 2-oxobutyle, ou le groupe

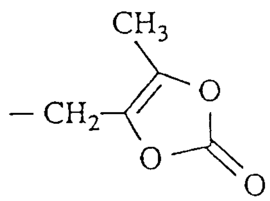

$R_2$ représente l'hydrogène ou un groupe méthyle, ou un groupe phényle ou thiényle éventuellement substitué par le chlore, le fluor, des groupes méthyles, hydroxy ou méthoxy,

$R_3$ représente l'hydrogène ou un groupe méthyle,

et leurs hydrates, sels formés par addition avec des acides, sels alcalins, alcalino-terreux, d'argent et de guanidinium acceptables pour l'usage pharmaceutique, ainsi que leurs esters méthyliques, éthyliques, pivaloyloxyméthyliques, pivaloyloxyéthyliques ou 5-méthyl-2-oxo-1,3-dioxole-4-yl-méthyliques, sous réserve que $R_1$ ne peut représenter un groupe méthyle lorsque X représente le fluor.

**4.** L'acide 1-cyclopropyl-6-fluoro-1,4-dihydro-7-(3-méthyl-1-pipérazinyl)-4-oxo-1,8-naphtyridine-3-carboxylique.

**5.** L'acide 1-cyclopropyl-6-fluoro-1,4-dihydro-7-[4-(2-hydroxyéthyl)-1-pipérazinyl]-4-oxo-1,8-naphtyridine-3-carboxylique.

**6.** Procédé de préparation des acides 1-cyclopropyl-1,4-dihydro-4-oxo-1,8-napthyridine-3-carboxyliques de la revendication 1, caractérisé en ce que l'on fait réagir des acides 1-cyclopropyl-7-halogéno-1,4-dihydro-4-oxo-1,8-naphtyridine-3-carboxyliques de formule II

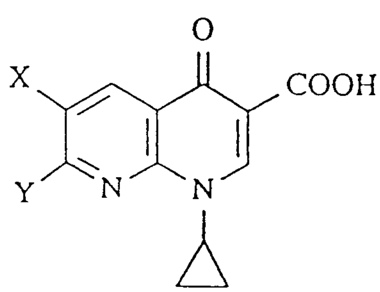

(II)

dans laquelle X a les significations indiquées ci-dessus et Y représente un halogène, de préférence le chlore ou le fluor, avec des amines de formule III

A - H (III)

dans laquelle A a les significations indiquées ci-dessus, éventuellement en présence d'accepteurs d'acides, et le cas échéant on convertit en les sels, esters et autres formes de "promédicaments".

**7.** Médicament contenant des acides 1-cyclopropyl-1,4-dihydro-4-oxo-1,8-naphtyridine-3-carboxyliques selon la revendication 1.

**8.** Utilisation des acides 1-cyclopropyl-1,4-dihydro-4-oxo-1,8-naphtyridine-3-carboxyliques de la revendication 1 pour la préparation de médicaments.

**Revendications pour l'Etat contractant suivant : AT**

**1.** Procédé de préparation des acides 1-cyclo-1,4-dihydro-4-oxo-1,8-naphtyridine-3-carboxyliques de formule I

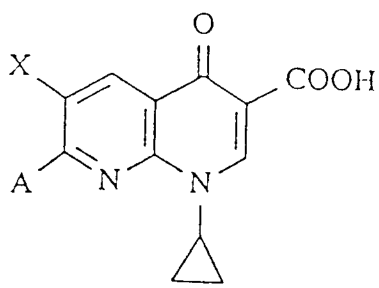

(I)

dans laquelle
X représente un halogène et
A représente

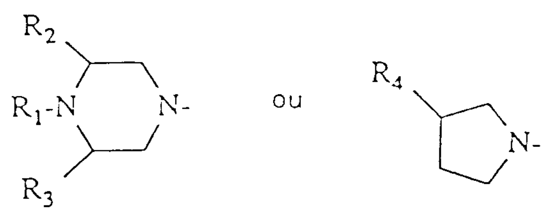

dans lesquels
$R_1$ représente un groupe alkyle à chaîne droite ou ramifiée en $C_1$-$C_4$ qui peut être substitué par un groupe hydroxy ou éthoxy, un groupe phénacyle éventuellement substitué par des groupes hydroxy, méthoxy, le chlore ou le fluor, un groupe 2-oxopropyle, 2-oxobutyle, ou le groupe

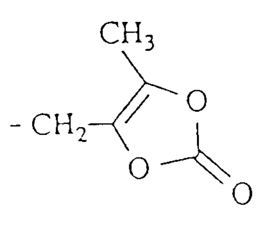

$R_2$ représente l'hydrogène ou un groupe méthyle, ou un groupe phényle ou thiényle éventuellement substitué par le chlore, le fluor, des groupes méthyle, hydroxy ou méthoxy,
$R_3$ représente l'hydrogène ou un groupe méthyle et
$R_4$ représente un groupe hydroxyméthyle
et de leurs hydrates, sels formés par addition avec des acides, sels alcalins, alcalino-terreux, d'argent et de guanidinium acceptables pour l'usage pharmaceutique, ainsi que de leurs esters méthyliques, éthyliques, pivaloyloxyméthyliques, pivaloyloxyéthyliques, ou 5-méthyl-2-oxo-1,3-dioxole-4-yle-méthyli-

ques, sous réserve que $R_1$ ne peut représenter un groupe méthyle lorsque X représente le fluor, caractérisé en ce que l'on fait réagir des acides 1-cyclopropyl-7-halogéno-1,4-dihydro-4-oxo-1,8-naphtyridine-3-carboxyliques de formule II

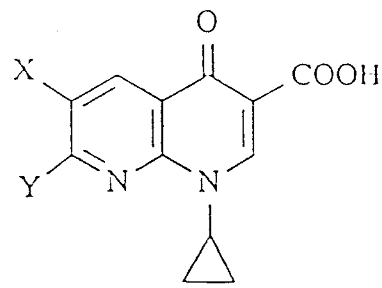

(II)

dans laquelle X a les significations indiquées ci-dessus et Y représente un halogène, de préférence le chlore ou le fluor, avec des amines de formule III

A - H (III)

dans laquelle A a les significations indiquées ci-dessus ; éventuellement en présence d'accepteurs d'acides, et le cas échéant on convertit en les sels, esters et autres formes de "promédicaments".

**2.** Procédé de préparation des acides 1-cyclopropyl-1,4-dihydro-4-oxo-1,8-naphtyridine-3-carboxyliques selon la revendication 1,
dans lesquels
X représente le fluor ou le chlore et
A représente

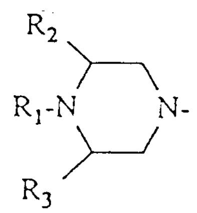

ou

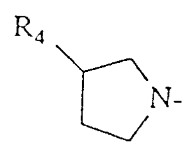

dans lesquels
$R_1$ représente un groupe alkyle à chaîne droite ou ramifiée en $C_1$-$C_4$ qui est substitué par un groupe hydroxy ou éthoxy, un groupe phénacyle substitué par un groupe hydroxy, méthoxy, le chlore ou le fluor, un groupe oxopropyle, 2-oxobutyle, ou le groupe

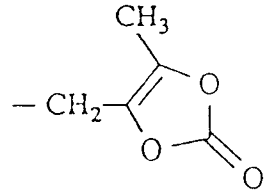

$R_2$ représente l'hydrogène ou un groupe méthyle, ou un groupe phényle ou thiényle éventuellement substitué par le chlore, le fluor, des groupes méthyle, hydroxy ou méthoxy,
$R_3$ représente l'hydrogène ou un groupe méthyle et
$R_4$ un groupe hydroxyméthyle,
et de leurs hydrates, sels formés par addition avec des acides, sels alcalins, alcalino-terreux, d'argent et de guanidinium acceptables pour l'usage pharmaceutique, ainsi que de leurs esters méthyliques, éthyliques, pivaloyloxymétyliques, pivaloyloxyéthyliques ou 5-méthyl-2-oxo-1,3-dioxole-4-yl-méthyli-

ques, sous réserve que $R_1$ ne peut représenter un groupe méthyle lorsque X représente le fluor, caractérisé en ce que l'on fait réagir des acides 1-cyclopropyl-7-halogéno-1,4-dihydro-4-oxo-1,8-naphtyridine-3-carboxyliques de formule II

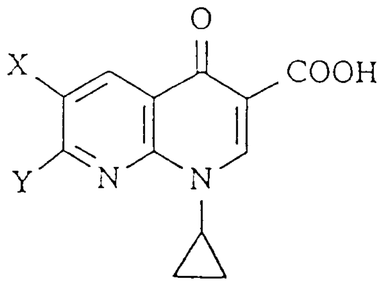

(II)

dans laquelle X a les significations indiquées ci-dessus et Y représente un halogène, de préférence le chlore ou le fluor, avec des amines de formule III

A - H (III)

dans laquelle A a les significations indiquées ci-dessus, éventuellement en présence d'accepteurs d'acides, et le cas échéant on convertit en les sels, esters et autres formes de "promédicaments".